# EUROPEAN PATENT APPLICATION

(11) **EP 1 521 080 A1**
(43) Date of publication of application: **06.04.2005**
(21) Application number: 04255871.8
(22) Date of filing: 25.09.2004
(51) Int. Cl.: G01N 33/00

(54) **Optical hydrogen sensor and system**

(30) Priority: 02.10.2003 JP 2003344766
(71) Applicant: ALPS ELECTRIC CO., LTD., Ota-ku Tokyo 145 (JP)
(72) Inventor: Fukuda, Kouichi, c/o Alps Electric Co.,Ltd., Ota-ku Tokyo (JP)
(74) Representative: Kensett, John Hinton

(57) **Abstract**

A hydrogen sensor (9) for determining the content of hydrogen based on the transmittance of light includes a substrate having an n-type semiconductor layer (2) placed thereon and also includes a metal layer (4) that is placed on the n-type semiconductor layer, forms a Schottky junction with n-type semiconductor layer, and contains a metal of which the transmittance is varied when the metal adsorbs hydrogen.

## Description

### BACKGROUND OF THE INVENTION

### 1. Field of the Invention

The present invention relates to a hydrogen sensor for determining the hydrogen content in the ambient atmosphere and also relates to a hydrogen detection system including the hydrogen sensor.

### 2. Description of the Related Art

Known practical hydrogen sensors use mechanisms for varying the electrical conductivity of metal oxide semiconductors such as SnO₂ and ZnO by allowing the semiconductors to adsorb reductive gas.

The hydrogen sensors have a safety problem and a reproducibility problem because they are heated to high temperature to detect the change in electrical conductivity due to gas adsorption.

For example, Japanese Examined Patent Application Publication No. 03-15975 (hereinafter referred to as Patent Document 1) discloses a hydrogen sensor, shown in FIG. 5, for detecting hydrogen gas. The hydrogen sensor is of an optical type and need not therefore be heated.

The hydrogen sensor includes an insulating substrate 30, a conductive layer 31 that contains indium oxide (In₂O₃) and functions as an electrode, a compound semiconductor layer 32 containing tungsten trioxide (WO₃), an electrode layer 33 containing palladium (Pd), a first electrode wire 34, and a second electrode wire 35, those layers being disposed on the insulating substrate 30 in that order.

Since palladium contained in the electrode layer 33 has a catalytic function, the electrode layer 33 adsorbs hydrogen molecules to dissociate the hydrogen molecules into hydrogen atoms. The dissociated hydrogen atoms are diffused in the compound semiconductor layer 32, whereby current-voltage characteristics of the compound semiconductor layer 32 are varied.

The hydrogen sensor determines the hydrogen content by measuring changes in the current-voltage characteristics with the first and second electrode wires 34 and 35.

The hydrogen sensor disclosed in Patent Document 1 has a problem in that the response speed is slow because the sensor measures changes in the current-voltage characteristics of the compound semiconductor layer 32, the changes being caused by the dissociated hydrogen atoms diffused in the compound semiconductor layer 32 as described above, and it takes a long time to diffuse and then release the dissociated hydrogen atoms.

The hydrogen sensor further has a problem in that WO₃ contained in the compound semiconductor layer 32 is sensitive to water and the detection accuracy thereof is therefore deteriorated in a short time.

Furthermore, the hydrogen sensor has a problem in that the sensor has low selective sensitivity to hydrogen and the accuracy of the hydrogen content is therefore low because the sensor is sensitive not only to hydrogen but also to hydrogen-containing compounds such as ammonia (NH₃) and hydrogen sulfide (H₂S).

### SUMMARY OF THE INVENTION

The present invention has been made to solve the problems described above. It is an object of the present invention to provide a hydrogen sensor having a high response speed, high water resistance, and high selective sensitivity to hydrogen and also provides a hydrogen detection system including the hydrogen sensor.

A hydrogen sensor of the present invention is used to determine the hydrogen content based on the transmittance of light. The hydrogen sensor includes a substrate having an n-type semiconductor layer placed thereon and also includes a metal layer that is placed on the n-type semiconductor layer, forms a Schottky junction with the n-type semiconductor layer, and contains a metal of which the transmittance is varied when the metal adsorbs hydrogen.

The hydrogen sensor uses a mechanism for generating photocarriers at the Schottky junction. When the metal layer adsorbs hydrogen, the transmittance of the metal layer is varied and the energy of light reaching the Schottky junction is therefore varied, whereby the amount of the generated photocarriers is varied.

When the light applied to the Schottky junction has a wavelength of 450 to 650 nm, the efficiency of generating photocarriers (electron-hole pairs) is high and the photocurrent density is therefore high, thereby achieving high measurement accuracy.

The hydrogen sensor of the present invention measures the hydrogen content based on the amount of the photocarriers generated at the Schottky junction in contrast to known hydrogen sensors that measure the hydrogen content based on changes in current-voltage characteristics due to the diffusion and release of hydrogen. Therefore, the hydrogen sensor can quickly respond to a change in hydrogen content.

The hydrogen sensor may further include an anti-reflective layer, placed on the metal layer, having a function of selectively allowing hydrogen molecules to pass therethrough.

Since the anti-reflective layer has such a function, water molecules having a molecular size greater than that of the hydrogen molecules cannot pass through the anti-reflective layer; hence, the hydrogen sensor has high water resistance.

Furthermore, since hydrogen compound gases such as NH₃ and H₂S cannot pass through the anti-reflective layer, the metal layer is not in contact with gases other than hydrogen; hence, the hydrogen content can be determined with high accuracy.

Since the anti-reflective layer is placed on the metal layer, incident light is not reflected but can pass through the metal layer to reach the Schottky junction efficiently. Therefore, the photocarriers can be efficiently generated, that is, a photocurrent can be efficiently generated; hence, the sensitivity of detecting hydrogen, or the sensitivity of measuring the hydrogen content is high.

In the hydrogen sensor, the metal is preferably any one of palladium, platinum, and rhodium.

Since the transmittance of the metal layer is effectively varied depending on the amount of hydrogen molecules adsorbed on the metal layer, the hydrogen content can be determined with high accuracy. When light having a wavelength of 450 to 650 nm is applied to the metal layer in particular, the transmittance is significantly varied. Therefore, the measurement accuracy can be enhanced by selecting a suitable wavelength.

In the hydrogen sensor, the anti-reflective layer preferably contains one of SiO₂ and AlOₓ. Therefore, the anti-reflective layer can be readily formed. Since the anti-reflective layer substantially prevents water, NH₃, and H₂S molecules from passing therethrough, the sensitivity of detecting hydrogen is high and the hydrogen sensor has high water resistance.

Furthermore, since the anti-reflective layer is placed on the metal layer, the metal layer can efficiently absorb light; hence, the measurement accuracy is high.

A hydrogen detection system of the present invention includes the hydrogen sensor having any one of the above configurations. Therefore, the hydrogen detection system can quickly respond to a change in hydrogen content and measure the hydrogen content with high accuracy.

As described above, the hydrogen sensor of the present invention measures the amount of the photocarriers generated at the Schottky junction to determine the hydrogen content, in contrast to known hydrogen sensors that measure changes in current-voltage characteristics due to the diffusion and release of hydrogen to determine the hydrogen content. Therefore, the hydrogen sensor can quickly respond to a change in hydrogen content.

In the hydrogen sensor, the anti-reflective layer, which selectively allows hydrogen molecules to pass therethrough, is placed on the metal layer. That is, the anti-reflective layer that prevents molecules having a size greater than that of a hydrogen molecule from passing therethrough is placed on the metal layer. Therefore, water molecules having a size greater than that of the hydrogen molecule cannot pass through the anti-reflective layer; hence, the hydrogen sensor has high water resistance.

Furthermore, since hydrogen compound gases such as NH₃ and H₂S cannot also pass through the anti-reflective layer, the metal layer is not in contact with gases other than hydrogen; hence, the hydrogen sensor can be used to determine the hydrogen content with high accuracy.

Embodiments of the invention will now be described, by way of example only, with reference to the accompanying drawings, in which:
FIG. 1 is a schematic sectional view showing a configuration of a first hydrogen sensor according to a first embodiment of the present invention;
FIG. 2 is a graph showing photocurrent density-voltage correlations of the first hydrogen sensor depending on the hydrogen content;
FIG. 3 is a schematic sectional view showing a configuration of a second hydrogen sensor according to a second embodiment of the present invention;
FIG. 4 is a schematic sectional view showing a configuration of a hydrogen detection system including a hydrogen sensor of the present invention; and

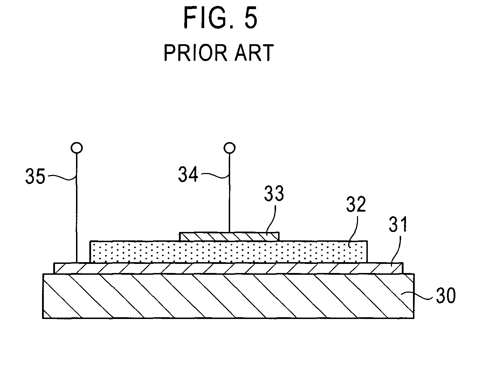

FIG. 5 is a schematic sectional view showing a known hydrogen sensor.

### DESCRIPTION OF THE PREFERRED EMBODIMENTS

### [First Embodiment]

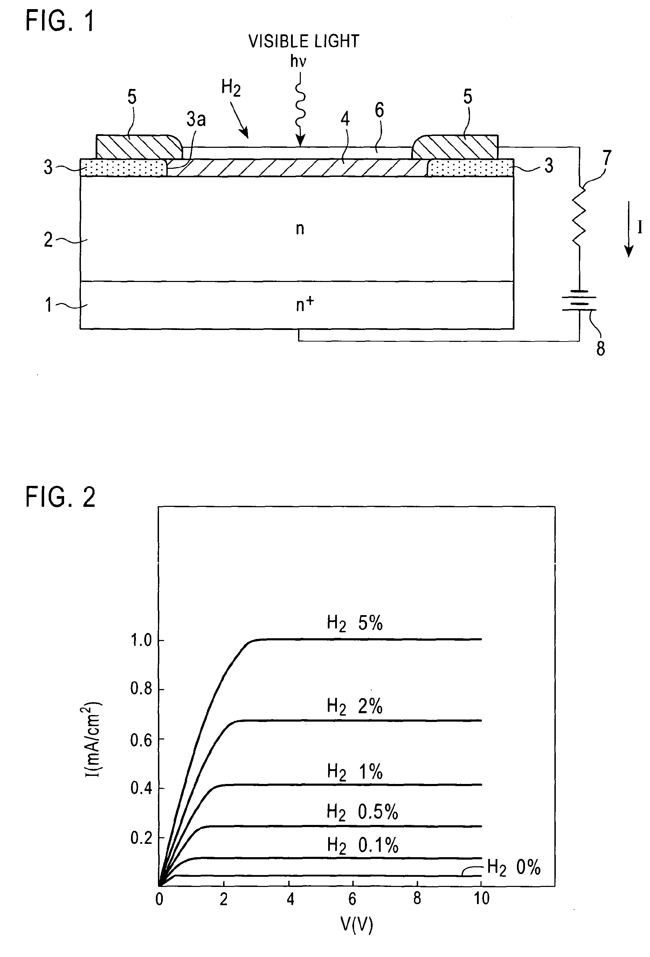

A first hydrogen sensor 9 according to a first embodiment of the present invention will now be described with reference to the accompanying drawings. FIG. 1 is a schematic sectional view showing a configuration of the hydrogen sensor. With reference to FIG. 1, an n-type semiconductor layer 2 is made of an n-type semiconductor doped with an n-type impurity and has a resistivity of, for example, five to ten Ωcm. An n⁺-type semiconductor layer 1 into which n-type impurity ions have been implanted at a dose of 10¹⁷/cm³ to 10¹⁸/cm³ is placed under the n-type semiconductor layer 2 and is in ohmic contact with a metal electrode. An insulating layer 3 is placed on the n-type semiconductor layer 2, contains, for example, SiO₂ or the like, and has an opening 3a, formed by an etching process, functioning as a detection section. The first hydrogen sensor 9 may be placed on an insulating substrate if the first hydrogen sensor 9 includes the n-type semiconductor layer 2 and the n⁺-type semiconductor layer 1 to which an extractor electrode can be fixed.

A metal layer 4 is semitransparent, has a thickness of about 10 nm, and contains a metal, such as palladium (Pd), platinum (Pt), or rhodium (Rh), having a catalytic function. The metal layer 4 adsorbs hydrogen molecules to dissociate the hydrogen molecules into hydrogen atoms and the transmittance of the metal layer 4 varies depending on the amount of the adsorbed hydrogen molecules. The metal layer 4 is in contact with the n-type semiconductor layer 2 and functions as an electrode for forming a Schottky junction with the n-type semiconductor layer 2.

An electrode layer 5 is electrically connected to the metal layer 4 and therefore functions as an extractor electrode for electrically connecting the metal layer 4 to an external component.

An anti-reflection layer 6 is made of, for example, a porous ceramic material and has a thickness of about 50 nm. Examples of the porous ceramic material include silicon dioxide (SiO₂) and alumina (AlOₓ). The anti-reflection layer 6 functions as a porous membrane that selectively allows molecules having a molecular size less than that of a hydrogen molecule to pass therethrough but prevents molecules of hydrogen compounds, such as ammonia (NH₃) and hydrogen sulfide (H₂S), having a molecular size greater than that of the hydrogen molecule from passing therethrough, that is, the anti-reflection layer 6 functions as a filter. Furthermore, the anti-reflection layer 6 prevents a water (H₂O) molecule from passing therethrough and therefore protects the metal layer 4 from moisture.

The operation of the first hydrogen sensor 9 will now be described with reference to FIG. 1.

A device, including the first hydrogen sensor 9, for measuring the photocurrent (I) created depending on the content of hydrogen gas is briefly described below.

A resistor 7 and a constant voltage power supply 8 are electrically connected to the first hydrogen sensor 9 in series and those components form a loop circuit. One end of the resistor 7 is electrically connected to the electrode layer 5 and the other one is electrically connected to the negative terminal of the constant voltage power supply 8. The positive terminal of the constant voltage power supply 8 is electrically connected to the n⁺-type semiconductor layer 1 with an extractor electrode placed therebetween.

That is, the constant voltage power supply 8 is electrically connected to the metal layer 4 with the resistor 7 and electrode layer 5 placed therebetween and also connected to the n⁺-type semiconductor layer 1 such that a reverse bias voltage is applied to the Schottky junction, that is, a Schottky diode consisting of the n-type semiconductor layer 2 and the metal layer 4.

Therefore, when the Schottky junction is irradiated with light, pairs of holes and electrons that are photocarriers are created. An electric field created at the junction separates the electron-hole pairs to inject the holes and the electrons into the metal layer 4 and the n-type semiconductor layer 2, respectively.

The holes and the electrons flow into the constant voltage power supply 8 for supplying the reverse bias voltage, whereby a photocurrent is generated. The potential difference between the ends of the resistor 7 is varied by the photocurrent.

The photocurrent varies depending on the energy of light applied to the Schottky junction.

The metal layer 4 has a catalytic function and therefore dissociates hydrogen gas, that is, hydrogen molecules adsorbed thereon, into hydrogen atoms, which are diffused in the metal layer 4. The transmittance of the metal layer 4 is varied by the hydrogen adsorption.

FIG. 2 shows that the photocurrent density varies depending on the hydrogen content when the energy of light applied to the first hydrogen sensor 9 is constant. This is because the amount of the adsorbed hydrogen molecules depends on the hydrogen content. With reference to FIG. 2, the vertical axis represents the density of the photocurrent flowing in the resistor 7 and the horizontal axis represents the voltage of the constant voltage power supply 8. Numbers placed each on corresponding photocurrent density-voltage curves represent the hydrogen content. An increase in the hydrogen content increases the photocurrent density.

### [Second Embodiment]

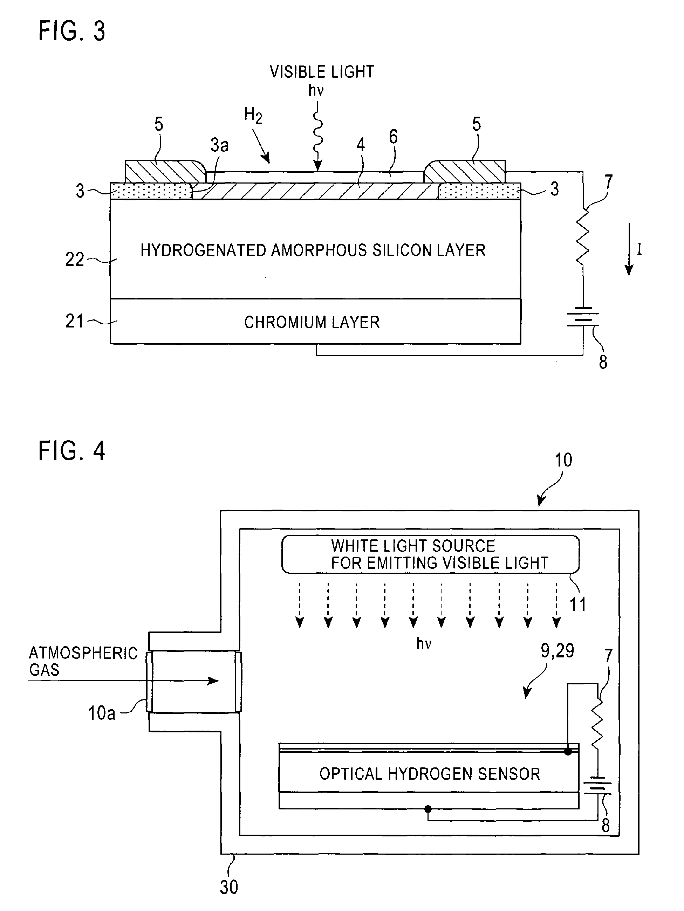

A second hydrogen sensor 29 according to a second embodiment of the present invention will now be described with reference to FIG. 3. In FIG. 3, the same components as those described in the first embodiment have the same reference numerals and descriptions of the components are omitted.

The second hydrogen sensor 29 includes a hydrogenated amorphous silicon (a-Si:H) layer 22 instead of the n-type semiconductor layer 2 described in the first embodiment. The hydrogenated amorphous silicon layer 22 has n-type semiconductor characteristics.

A chromium (Cr) layer 21 is placed under the hydrogenated amorphous silicon layer 22 such that the hydrogenated amorphous silicon layer 22 and an extractor electrode form an ohmic junction. The chromium layer 21 can be formed by a sputtering process, a vapor deposition process, or another process.

In the above configuration, the hydrogenated amorphous silicon layer 22 is in contact with a metal layer 4 and forms a Schottky junction with the metal layer 4.

Other components and the operation of the second hydrogen sensor 29 are the same as those of the first hydrogen sensor 9 of the first embodiment; hence, descriptions of the components and the operation are omitted.

### [Hydrogen Detection System]

A hydrogen detection system 10 including the first or second hydrogen sensor 9 or 29 according to the first or second embodiment, respectively, will now be described with reference to FIG. 4. FIG. 4 is a schematic view showing a configuration of the hydrogen detection system 10.

A white light source 11 emits visible light with a predetermined energy. The first or second hydrogen sensor 9 or 29 is placed at a predetermined position at a predetermined angle such that the visible light emitted from the white light source 11 is incident on the opening 3a. The hydrogen detection system 10 includes a housing 30 for storing the first or second hydrogen sensor 9 or 29. The housing 30 is made of a material that can prevent light from passing therethrough; hence, the first or second hydrogen sensor 9 or 29 is shielded from external light.

The housing 30 has an air intake 10a through which a predetermined amount of air is taken in with a motor, which is not shown, in a unit time. A filter for trapping dust is placed in the air intake 10a, whereby the opening 3a of the first or second hydrogen sensor 9 or 29 is protected from dust.

Measurement is performed using a controller as described below. The relationship between the hydrogen content and the potential difference between the ends of the resistor 7 is determined in advance. A formula or a look-up table is prepared based on the relationship and then stored in a memory. During the measurement of the hydrogen content, the controller detects the potential difference between the ends of the resistor 7 and then calculates the hydrogen content using the formula or reads a hydrogen content corresponding to the potential difference from the look-up table, thereby obtaining the hydrogen content in the atmosphere of a place at which the hydrogen detection system 10 is placed.

## Claims

1. A hydrogen sensor for determining the hydrogen content based on the transmittance of light, comprising:
a substrate including an n-type semiconductor layer placed thereon; and
a metal layer that is placed on the n-type semiconductor layer, forms a Schottky junction with the n-type semiconductor layer, and contains a metal of which the transmittance is varied when the metal adsorbs hydrogen.

2. The hydrogen sensor according to Claim 1 further comprising an anti-reflective layer, placed on the metal layer, having a function of selectively allowing hydrogen molecules to pass therethrough.

3. The hydrogen sensor according to Claim 1, wherein the metal is any one of palladium, platinum, and rhodium.

4. The hydrogen sensor according to Claim 2, wherein the metal is any one of palladium, platinum, and rhodium.

5. The hydrogen sensor according to Claim 2, wherein the anti-reflective layer contains one of SiO₂ and AlOₓ.

6. A hydrogen detection system comprising the hydrogen sensor according to any Claim 1 to 5.
